# EUROPÄISCHE PATENTANMELDUNG

(11) **EP 3 797 814 A1**
(43) Veröffentlichungstag der Anmeldung: **31.03.2021**
(21) Anmeldenummer: 20197530.7
(22) Anmeldetag: 22.09.2020
(51) Int. Cl.: A61M 15/08, A61M 11/04, A62B 23/06

(54) **MINIATURISIERTES INHALATIONSGERÄT**

(30) Priorität: 26.09.2019 DE 102019126003
(71) Anmelder: Aspuraclip GmbH, 12529 Schönefeld (DE)
(72) Erfinder: KLEINER, Wolfgang, 12529 Berlin (DE); WOLLMERSHÄUSER, Manfred, 12529 Berlin (DE); TIET, Vinh-Nghi, 12529 Berlin (DE)
(74) Vertreter: Bittner, Thomas L.

(57) **Zusammenfassung**

Die Erfindung betrifft ein miniaturisiertes Inhalationsgerät (1) zum Inhalieren mindestens eines Wirkstoffs durch die Nase mit: einer im Wesentlichen U-förmigen Bauteilform und einem ersten und zweiten länglichen Speicherabschnitt (2, 3), welche jeweils einen Hohlraum (9, 10) zum Aufnehmen eines Wirkstoffs aufweisen und eingerichtet sind, jeweils in ein Nasenloch eingeführt zu werden. Die Hohlräume (9, 10) weisen jeweils eine deckseitige Öffnung (2c, 3c) auf, über die der Wirkstoff aus dem entsprechenden Hohlraum (9, 10) austreten kann. Das Inhalationsgerät (1) weist ferner einen gekrümmten Bügelabschnitt (4) auf, welcher den ersten und den zweiten länglichen Speicherabschnitt (2, 3) einstückig verbindet und sich zumindest in einem Fußbereich der U-förmigen Bauteilform erstreckt, und einen Selbsthaltemechanismus zum Halten an der Nase, bei dem der erste und der zweite längliche Speicherabschnitt (2, 3) mittels des gekrümmten Bügelabschnitts (4) gegen ein den Abstand zwischen dem ersten und dem zweiten länglichen Speicherabschnitt (2, 3) vergrößerndes Aufbiegen elastisch vorgespannt sind und im Bereich des ersten und des zweiten länglichen Speicherabschnitts (2, 3) jeweils innenseitig ein einstückig angeformter Klemmvorsprung (12, 13) angeordnet ist, welche zum Halten auf gegenüberliegenden Seiten der Nasenscheidewand zur Anlage kommen. Es kann ferner ein Set mit zumindest einem Inhalationsgerät (1) und einem Aufbewahrungsbehältnis (20) vorgesehen sein.

## Beschreibung

Die Erfindung betrifft ein miniaturisiertes Inhalationsgerät zum Inhalieren mindestens eines Wirkstoffs durch die Nase.

### Hintergrund

Ein miniaturisiertes Inhalationsgerät zum Inhalieren eines Wirkstoffs durch die Nase ist aus dem Dokument DE 20 2011 102 694 U1 bekannt. Das Inhalationsgerät in Miniaturformat besteht aus einem Gehäuse und einem Wirkstoffspeicher. Das Gehäuse, dessen größerer Teil innerhalb der Nase platziert werden soll, ist aus einem elastischen U-förmigen Röhrchen, in dem sich Hohlräume befinden, die mit einem Wirkstoffspeicher aus einem faserigen oder porösen Material gefüllt sind.

Miniaturisierte Inhalationsgeräte sind weiterhin in den Dokumenten DE 10 2014 014 684 A1 sowie DE 20 2013 000 744 U1 beschrieben.

### Zusammenfassung

Aufgabe der Erfindung ist es, ein miniaturisiertes Inhalationsgerät zum Inhalieren mindestens eines Wirkstoffs durch die Nase (Naseninhalation oder -atmung) anzugeben, welches auf verbesserte Art und Weise für die Inhalation des Wirkstoffs durch die Nase eingerichtet ist, insbesondere hinsichtlich der Bereitstellung des zu inhalierenden Wirkstoffs sowie eines Selbsthalts des Geräts an der Nase.

Zur Lösung ist ein miniaturisiertes Inhalationsgerät zum Inhalieren mindestens eines Wirkstoffs durch die Nase nach dem unabhängigen Anspruch 1 geschaffen. Ausgestaltungen sind Gegenstand von abhängigen Unteransprüchen.

Nach einem Aspekt ist ein miniaturisiertes Inhalationsgerät zum Inhalieren mindestens eines Wirkstoffs durch die Nase geschaffen, welche eine im Wesentlichen u-förmigen Bauteilform aufweist und mit folgenden Merkmalen gebildet ist: einem ersten länglichen Speicherabschnitt, welcher einen ersten Hohlraum zum Aufnehmen einer ersten Menge eines Wirkstoffs aufweist und eingerichtet ist, in ein erstes Nasenloch eingeführt zu werden, wobei der erste Hohlraum einen erste deckseitige Öffnung aufweist, über die der Wirkstoff aus dem ersten Hohlraum austreten kann; einem zweiten länglichen Speicherabschnitt, welcher einen zweiten, von dem ersten Hohlraum getrennten Hohlraum zum Aufnehmen einer zweiten Menge des Wirkstoffs oder eines anderen Wirkstoffs aufweist und eingerichtet ist, in ein zweites Nasenloch eingeführt zu werden, wobei der zweite Hohlraum einen zweite deckseitige Öffnung aufweist, über die der Wirkstoff und / oder der andere Wirkstoff aus dem zweiten Hohlraum austreten kann; einem gekrümmten Bügelabschnitt, welcher den ersten und den zweiten länglichen Speicherabschnitt einstückig verbindet und sich zumindest in einem Fußbereich der u-förmigen Bauteilform erstreckt; und einem Selbsthaltemechanismus zum Halten an der Nase, bei dem der erste und der zweite längliche Speicherabschnitt mittels des gekrümmten Bügelabschnitts gegen ein den Abstand zwischen dem ersten und dem zweiten länglichen Speicherabschnitt vergrößerndes Aufbiegen elastisch vorgespannt sind und im Bereich des ersten und des zweiten länglichen Speicherabschnitts jeweils innenseitig ein einstückig angeformter Klemmvorsprung angeordnet ist, welche zum Halten auf gegenüberliegenden Seiten der Nasenscheidewand zur Anlage kommen.

Der Wirkstoff ist in den Hohlräumen in flüssiger Form (Flüssigkeitsmenge oder -probe) bereitgestellt und kann so durch die jeweilige Hohlraumöffnung hindurch inhaliert werden.

Der erste und der zweite längliche Speicherabschnitt sowie der gekrümmte Bügelabschnitt können einen einstückigen Bauteilkörper mit der u-förmigen Bauteilform bilden, welcher aus einem Kunststoffmaterial besteht. Als Kunststoffmaterial kann zum Beispiel ein Harzmaterial oder ein Elastomer verwendet werden. Hierbei kommen zum Beispiel Kunststoffmaterialien zum Einsatz, die für die Verwendung im Zusammenhang mit Lebensmitteln zugelassen sind.

Der gekrümmte Bügelabschnitt kann im Querschnitt flach ausgebildet sein. Das Flachmaterial kann frei von Hohlräumen sein. Insbesondere weist das Flachmaterial keine Hohlräume auf, die eine Fluidverbindung zwischen dem ersten und dem zweiten Hohlraum bilden. Diese sind in den verschiedenen Ausführungsformen stets voneinander getrennt, ohne dass zwischen dem ersten und dem zweiten Hohlraum in dem ersten / zweiten länglichen Speicherabschnitt eine Fluidverbindung bestehen würde.

Die Anformung des gekrümmten Bügelabschnitts an den ersten und den zweiten länglichen Speicherabschnitt kann innenseitig erfolgen, zum Beispiel über einen sich zur Innenseite hin verjüngenden Abschnitt unterhalb des ersten / zweiten Hohlraums. Dieser sich zur Innenseite hin verjüngende Abschnitt ist sowohl mit dem länglichen Speicherabschnitt wie auch mit einem zugehörigen Ende des gekrümmten Bügelabschnitts einstückig verbunden. In alternativen Ausgestaltungen kann sich der Abschnitt zwischen dem länglichen Speicherabschnitt und dem gekrümmten Bügelabschnitt zur Mitte oder zur Außenseite hin verjüngen, so dass der Bügelabschnitt mittig oder außenseitig an den jeweiligen länglichen Speicherabschnitt einstückig anformt. Charakteristisch ist die Verjüngung von dem länglichen Speicherabschnitt zum gekrümmten Bügelabschnitt hin, welcher dann mit der Flachform oder einer anderen Form ausgebildet ist, die gegenüber den länglichen Speicherabschnitten eine verminderte Bauteildicke aufweist. Bei diesen oder anderen Ausführungsformen kann der einstückige Bauteilkörper des miniaturisierten Inhalationsgeräts als Spritzgussbauteil aus dem Kunststoffmaterial gebildet sein.

Der gekrümmte Bügelabschnitt kann an den ersten und den zweiten längliche Speicherabschnitt jeweils innenseitig einstückig angeformt sein.

Der erste und der zweite längliche Speicherabschnitt können jeweils eine Zylinderform aufweisen, zum Beispiel eine Rundzylinderform.

Der erste und der zweite Hohlraum können jeweils eine Zylinderform aufweisen. Der erste und der zweite Hohlraum können mit als Hohlräume mit einer runden Zylinderform ausgeführt sein.

Bei dem jeweiligen Klemmvorsprung kann eine Vorsprungspitze abgerundet sein. Alternativ oder ergänzend kann vorgesehen sein, dass die beidseitig der Vorsprungspitze des Klemmvorsprungs angeordneten Übergänge zwischen dem Klemmvorsprung und den länglichen Speicherabschnitt jeweils gerundet ausgebildet sind, zumindest mit Blick von vorn auf die u-förmige Bauteilform.

Der jeweilige Klemmvorsprung kann mit Blick von vorn auf die u-förmige Bauteilform eine Dreiecksform aufweisen. Alternativ kann eine Halbkreisform oder eine andere Mehreckform vorgesehen sein, bevorzugt jeweils mit abgerundeten Ecken.

Der jeweilige Klemmvorsprung kann in Längsrichtung des ersten / zweiten länglichen Speicherabschnitts mit diesem vollständig überlappend gebildet oder angeordnet sein. Hierbei können der jeweilige Klemmvorsprung und der erste / zweite längliche Speicherabschnitt im Wesentlichen eine gleiche Länge in Längsrichtung aufweisen. Auch kann vorgesehen sein, dass der Klemmvorsprung in Längsrichtung kürzer als der erste / zweite längliche Speicherabschnitt ist.

Der jeweilige Klemmvorsprung kann mit Blick von oben auf die u-förmige Bauteilform eine geringere Bauteildicke aufweisen als der erste / zweite längliche Speicherabschnitt. Alternativ kann der jeweilige Klemmvorsprung mit einer im Wesentlichen gleichen Bauteildicke wie der zugeordnete längliche Speicherabschnitt ausgeführt sein. Bei dieser oder anderen Ausführungsformen kann der jeweilige Klemmvorsprung mit einer im Wesentlichen vollständig abgerundeten Oberfläche ausgebildet sein.

Der jeweilige Klemmvorsprung kann unterhalb und beabstandet von einem umlaufenden Rand der ersten / zweiten deckseitigen Öffnung angeordnet sein. Alternativ kann sich ein fußseitiger Ausläufer des Klemmvorsprungs bis hin zum umlaufenden Rand der zugeordneten deckseitigen Öffnung erstrecken, beispielsweise am Rand vollständig auslaufend.

Es kann vorgesehen sein, dass der erste Hohlraum mit einem ersten Verschluss aus für den Wirkstoff durchlässigem Material versehen ist und der zweite Hohlraum mit einem zweiten Verschluss aus für den Wirkstoff und / oder den anderen Wirkstoff durchlässigem Material versehen ist. Um die Freigabe des Wirkstoffs zum Inhalieren in den Nasenlöchern zu gewährleisten, ist das Material für den ersten und den zweiten Verschluss, bei dem es sich um das gleiche Material oder unterschiedliche Materialien handeln kann, für den Wirkstoff oder die Wirkstoffe durchlässig, insbesondere in verdampfter oder flüchtiger Form. Beispielsweise kann es sich um ein poröses oder faserartiges Material handeln. Bevorzugt sind die mit dem jeweiligen Verschluss verschlossenen Hohlräume (außerhalb des Verschlusses selbst) frei von einem hierin angeordneten Einsatz, beispielsweise einem Materialeinsatz, welcher den Wirkstoff in aufgesaugter Form enthält (Wirkstoffspeicher).

Der erste und der zweite Verschluss können in ihrer Erstreckung in Längsrichtung des ersten / zweiten länglichen Speicherabschnitts auf einen Bereich oberhalb des jeweiligen Klemmvorsprungs begrenzt sein. Hierbei überlappen der jeweilige Klemmvorsprung und der im zugeordneten länglichen Speicherabschnitt angeordnete Verschluss in Längsrichtung des länglichen Speicherabschnitts im Wesentlichen nicht. Dieses bedeutet, dass der Verschluss vollständig oberhalb des Klemmvorsprungs angeordnet ist.

Der erste und der zweite Verschluss können gegenüber der ersten und der zweiten deckseitigen Öffnung zurückversetzt angeordnet sein. Der jeweilige Verschluss kann um die Dicke der Randwulst teilweise oder vollständig zurückversetzt sein. Alternativ kann vorgesehen sein, dass der Verschluss gegenüber der deckseitigen Öffnung um einen Abstand zurückversetzt ist, der größer als die Dicke der Randwulst ist.

In dem ersten und / oder dem zweiten Hohlraum kann ein Stopfen angeordnet sein, in welchem der Wirkstoff und / oder der andere Wirkstoff abgebbar aufgenommen sind. Hierbei kann der zum Beispiel lösbar in den zugeordneten Hohlraum eingebrachte Stopfen den Hohlraum ganz oder teilweise ausfüllen. Der Stopfen kann ganz oder teilweise aus einem saugfähigen Material sein, welches den jeweiligen Wirkstoff so aufgesaugt enthält, dass er bei der Nutzung dann über die Öffnung des Hohlraums entweichen kann. Beispielsweise kann es sich um ein poröses oder faserartiges Material handeln.

Der Selbsthaltemechanismus kann entlang des umlaufenden Randes der ersten und der zweiten deckseitigen Öffnung jeweils eine außenseitige Randwulst aufweisen. Die außenseitige Randwulst kann um die deckseitige Öffnung herum durchgehend oder unterbrochen ausgebildet sein. Die Randwulst kann eine gerundete Außenfläche aufweisen.

Die erste und die zweite deckseitige Öffnung können einen Öffnungsquerschnitt aufweisen, welcher im Wesentlichen gleich einem Hohlraumquerschnitt im Bereich des ersten und des zweiten Hohlraums ist. Alternativ kann der Öffnungsquerschnitt der deckseitigen Öffnung größer als der Hohlraumquerschnitt sein. Bei dieser Ausführungsform kann der Verschluss in einem Abschnitt mit dem größeren Öffnungsquerschnitt angeordnet sein. Am Übergang zwischen den verschiedenen Öffnungsquerschnitten kann eine Stufe als Anschlag für den Verschluss vorgesehen sein.

Gemäß einem Aspekt kann ein Aufbewahrungsbehältnis für das Inhalationsgerät vorgesehen sein. Demnach kann ein Set bereitgestellt werden, welches mindestens ein miniaturisiertes Inhalationsgerät wie oben beschrieben sowie ein Aufbewahrungsbehältnis aufweist. Vorteilhaft ist das Aufbewahrungsbehältnis eingerichtet, das Inhalationsgerät darin aufzunehmen, wobei das Aufbewahrungsbehältnis eine erste Aufnahme und eine zweite Aufnahme aufweist, welche bemessen und geformt sind, den ersten länglichen Speicherabschnitt bzw. den zweiten länglichen Speicherabschnitt jeweils zumindest teilweise darin aufzunehmen, sodass der erste und zweite Hohlraum gegenüber der Umgebung abgedichtet sind.

Abgedichtet bedeutet dabei insbesondere, dass der Wirkstoff oder die Wirkstoffe in gasförmiger bzw. flüchtiger Form nicht in die Umgebung gelangen, sobald das Inhalationsgerät in das Aufbewahrungsbehältnis eingeführt und darin bei Nicht-Nutzung aufbewahrt wird. Auf diese Weise kann die Haltbarkeit verlängert werden, da durch die Abdichtung bei Nicht-Nutzung ein Verflüchtigen des Wirkstoffs verhindert oder zumindest reduziert werden kann.

Das Aufbewahrungsbehältnis kann demnach auch als "Duft-Etui" bezeichnet werden. Vor der erstmaligen Nutzung des Inhalationsgeräts ist es dementsprechend auch vorteilhaft, dieses beispielsweise versiegelt bereitzustellen. Es kann ein Set mit mehreren (gleichen oder verschiedenen) Inhalationsgeräten und einem Aufbewahrungsbehältnis bereitgestellt werden.

Zur Abdichtung kann einfach vorgesehen sein, dass die jeweiligen deckseitigen Öffnungen gegen eine stirnseitige Wand der jeweiligen Aufnahme gedrückt und dadurch abgedichtet werden, beispielsweise wenn ein Deckel des Behältnisses geschlossen wird und das Inhalationsgerät dadurch in eine Endposition gedrückt bzw. zumindest in dieser gehalten wird. Weiterhin (oder auch alternativ) sind die Aufnahmen vorzugsweise derart gestaltet, dass durch die oben beschriebene umlaufende Randwulst eine entsprechende Dichtung gebildet wird. Mit anderen Worten, ein Innendurchmesser der Aufnahmen, insbesondere in einem Endbereich, in welchem die jeweilige Randwulst liegt, wenn der Inhalator in dem Aufbewahrungsbehältnis aufbewahrt wird, ist vorzugsweise gleich einem Außendurchmesser der Randwulst oder aber etwas kleiner, um ein Andrücken der Randwulst an die Innenwand der Aufnahmen und dadurch eine verbesserte Dichtung zu erreichen.

Das Aufbewahrungsbehältnis weist vorteilhaft einen Deckel auf, beispielsweise einen Klappdeckel, welcher zum Verschließen einrasten kann. Beliebige andere Deckelgestaltungen sind jedoch denkbar. Durch Verschließen des Deckels wird ein Herausfallen des Inhalators verhindert. Es kann jedoch auch vorgesehen sein, dass das Aufbewahrungsbehältnis mittels des Deckels derart verschlossen werden kann, dass eine weitere Abdichtung gegenüber der Umgebung gebildet wird. Darüber hinaus kann das Aufbewahrungsbehältnis weitere Merkmale aufweisen, wie beispielsweise eine Öse zum Befestigen an einem Schlüsselbund, einer Kette und dergleichen.

### Beschreibung von Ausführungsbeispielen

Im Folgenden werden weitere Ausführungsbeispiele unter Bezugnahme auf die Zeichnungen erläutert. Hierbei zeigen:
- Fig. 1: eine schematische perspektivische Darstellung eines miniaturisierten Inhalationsgeräts von vorn;
- Fig. 2: eine schematische perspektivische Darstellung des miniaturisierten Inhalationsgeräts aus Fig. 1 von schräg vorn;
- Fig. 3: eine schematische Darstellung einer Ausführung eines miniaturisierten Inhalationsgeräts im Schnitt;
- Fig. 4: eine schematische perspektivische Darstellung des miniaturisierten Inhalationsgeräts aus Fig. 3;
- Fig. 5: eine schematische perspektivische Darstellung eines Aufbewahrungsbehältnisses von schräg oben;
- Fig. 6: eine schematische perspektivische Darstellung des Aufbewahrungsbehältnisses aus Fig. 5 von schräg unten; und
- Fig. 7: eine schematische Darstellung des Aufbewahrungsbehältnisses aus Fig. 5 im Schnitt.

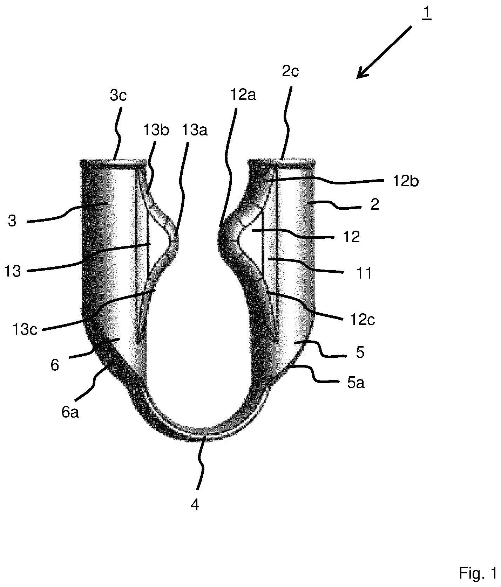

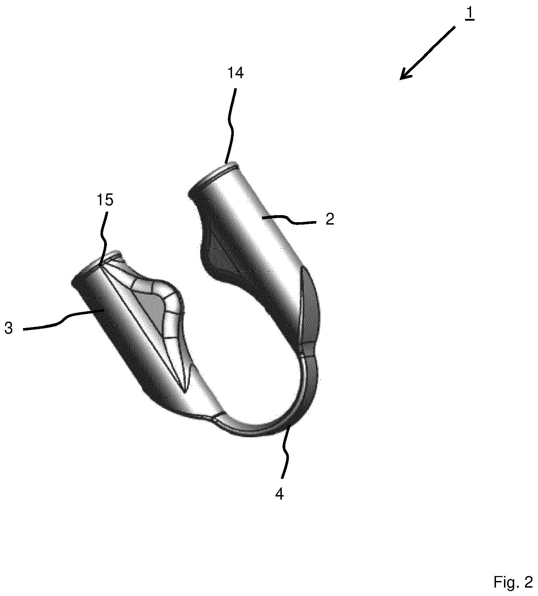

Die Fig. 1 und 2 zeigen perspektivische Darstellungen eines miniaturisierten Inhalationsgeräts 1, welches einen ersten und einen zweiten länglichen Speicherabschnitt 2, 3 aufweist, die über einen gekrümmten Bügelabschnitt 4 einstückig miteinander verbunden sind. Der gekrümmte Bügelabschnitt 4 weist in der gezeigten Ausführungsform einen flachen Querschnitt auf. Der gekrümmte Bügelabschnitt 4 erstreckt sich zumindest in einem unteren Bereich der u-förmigen Bauteilform des miniaturisierten Inhalationsgeräts 1.

An den ersten und den zweiten länglichen Speicherabschnitt 2, 3 ist der gekrümmte Bügelabschnitt 4 jeweils innenseitig einstückig angeformt. Dieses erfolgt an einem jeweiligen sich zum gekrümmten Bügelabschnitt 4 hin verjüngenden Abschnitt 5, 6. In dem gezeigten Ausführungsbeispiel bildet der sich verjüngende Abschnitt 5, 6 einen unteren Abschnitt 2a, 3a der länglichen Speicherabschnitte 2, 3 und ist auf einer Außenseite 5a, 6a abgeflacht.

Die länglichen Speicherabschnitte 2, 3 weisen eine Rundzylinderform auf. Am oberen Ende 2b, 3b ist eine jeweilige deckseitige Öffnung 2c, 3c vorgesehen, durch die jeweils ein Wirkstoff zum Inhalieren entweichen kann, wenn das miniaturisierte Inhalationsgerät 1 mit den länglichen Speicherabschnitten 2, 3 zumindest teilweise in eine Nase eingeführt ist (nicht dargestellt).

Der zu inhalierende Wirkstoff ist in Hohlräumen 9, 10 aufgenommen, die in den länglichen Speicherabschnitten 2, 3 angeordnet sind, zum Beispiel in Form zylindrischer Hohlräume, und mit den Öffnungen 2c, 3c in Fluidverbindungen stehen. Im jeweiligen Hohlraum 9, 10 kann ein Stopfen angeordnet sein, welcher den Hohlraum 9, 10 ganz oder teilweise ausfüllt (nicht dargestellt). Der Stopfen kann ganz oder teilweise aus einem saugfähigen Material sein, welches den jeweiligen Wirkstoff so aufgesaugt enthält, dass er bei der Nutzung dann über die Öffnung 2c, 3c des Hohlraums 9, 10 entweichen kann.

Zum Selbsthalten des miniaturisierten Inhalationsgeräts 1 an einer Nase ist ein Klemmmechanismus 11 mit Klemmvorsprüngen 12, 13 vorgesehen, die an den länglichen Speicherabschnitten 2, 3 innenseitig und einander gegenüberliegend angeordnet sind. Bei der gezeigten Ausführungsform weisen die Klemmvorsprünge 12, 13 mit Blick von vorn auf das miniaturisierte Inhalationsgerät 1 eine im Wesentlichen dreiecksförmige Gestaltung auf, wobei eine Vorsprungspitze 12a, 13a abgerundet ist, was auch für Übergänge 12b, 12c, 13b, 13c der Klemmvorsprünge 12, 13 zu den länglichen Speicherabschnitten 2, 3 hin gilt.

Die Klemmvorsprünge 12, 13 sind im Bereich der Hohlräume 9, 10 und unterhalb einer Randwulst 14, 15 angeordnet, die die jeweilige deckseitige Öffnung 2c, 3c umgibt.

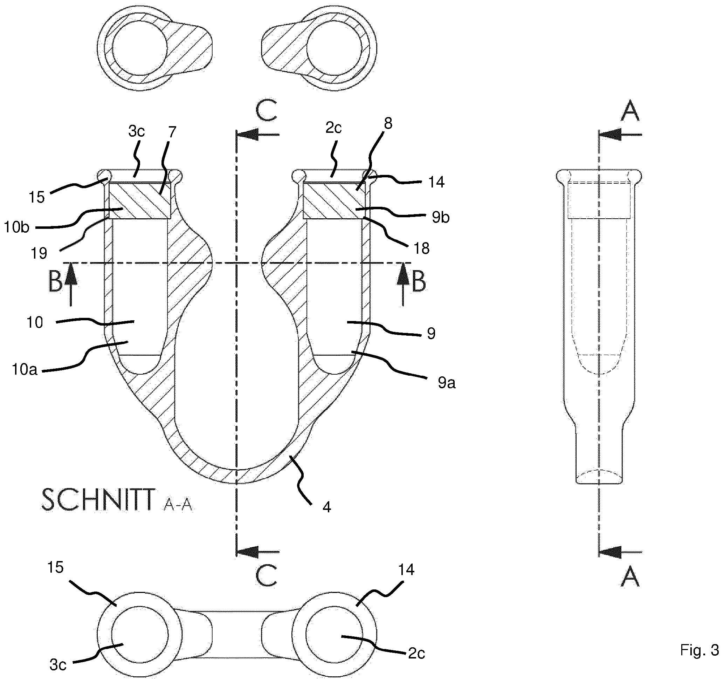

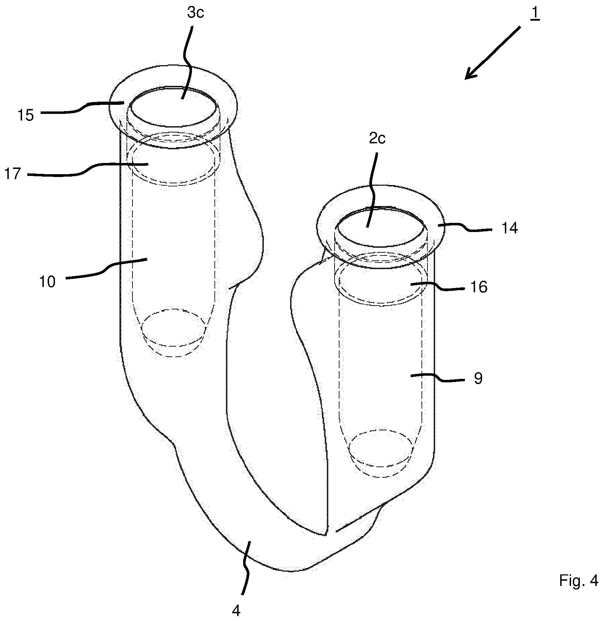

Die Fig. 3 und 4 zeigen eine weitere Ausführungsform eines miniaturisierten Inhalationsgeräts im Schnitt sowie perspektivisch. Für gleiche Merkmale werden dieselben Bezugszeichen wie in Verbindung mit den Fig. 1 und 2 verwendet. Die deckseiteigen Öffnungen 2c, 3c sind von der dichtenden Randwulst 14, 15 umgeben. Die Hohlräume 9, 10 verjüngen sich in einem unteren Abschnitt 9a, 10a. In einem oberen Abschnitt 9b, 10b ist ein Erweiterungsbereich 16, 17 gebildet, welcher der Aufnahme eines jeweiligen Verschlusses 7, 8 dient. Die Verschlüsse 7, 8 sind zum Inhalieren für den jeweiligen abzugebenden Wirkstoff durchlässig. Der zu inhalierende Wirkstoff kann dann in flüssiger Form in den Hohlräumen 9, 10 aufgenommen sein oder wie oben beschrieben aufgesaugt in einem Stopfen. Im Erweiterungsbereich 16, 17 ist die außen umlaufende Wandung jeweils verdünnt.

Am Übergang zwischen dem unteren Abschnitt 9a, 10a und dem jeweiligen oberen Abschnitt 9b, 10b, bzw. am unteren Ende des jeweiligen Erweiterungsbereichs 16, 17, ist in dem dargestellten Ausführungsbeispiel eine Stufe 18, 19 gebildet, die einen Anschlag für den jeweiligen Verschluss 7, 8 definiert. Dadurch wird verhindert, dass die Verschlüsse 7, 8 zu tief in den jeweiligen Hohlraum 9, 10 gelangen, insbesondere in den unteren Abschnitt 9a, 10a, welcher für den flüssigen Wirkstoff vorgesehen bzw. mit diesem gefüllt ist.

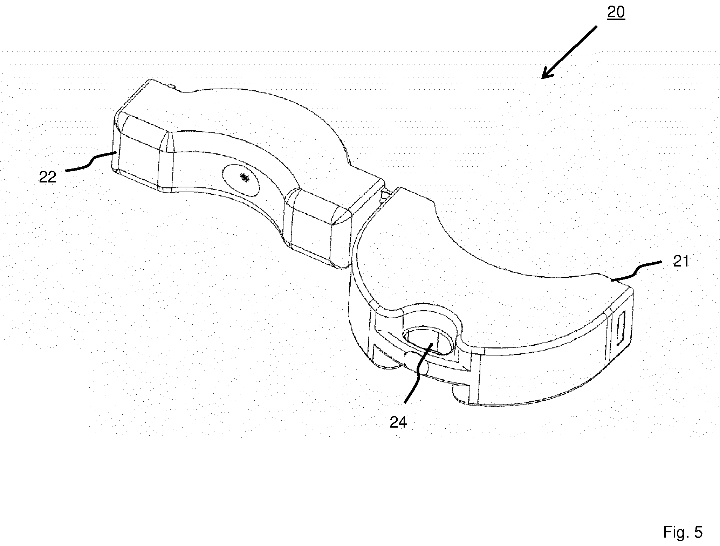

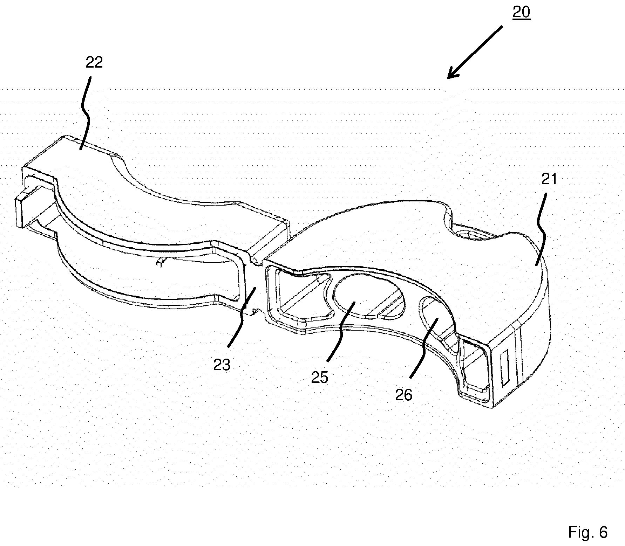

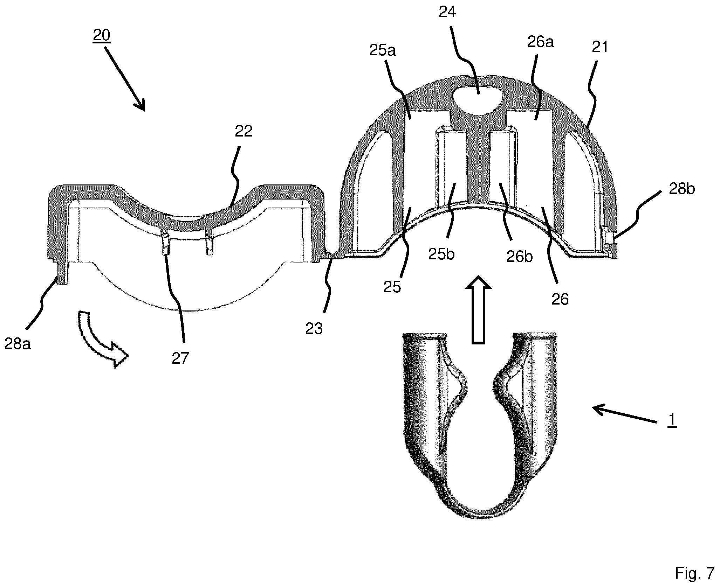

Die Fig. 5 und 6 zeigen perspektivische Darstellungen eines Aufbewahrungsbehältnisses 20. In Fig. 7 ist dieses im Schnitt und zusammen mit einem Inhalationsgerät 1 dargestellt. Das Inhalationsgerät 1 kann insbesondere bei Nicht-Nutzung und nach einem ersten Öffnen einer versiegelten Verpackung in dem Behältnis 20 aufbewahrt werden, um das Inhalationsgerät 1 zu schützen und dessen Haltbarkeit zu verbessern, beispielsweise einen Duft des Wirkstoffes zu bewahren (daher auch als "Duft-Etui" bezeichnet). Dazu wird das Inhalationsgerät 1 in einen Aufnahmeteil 21 des Behältnisses 20 eingeführt und der Deckel 22 geschlossen (vgl. entsprechende Pfeile in Fig. 7). In diesem Ausführungsbeispiel ist der Deckel 22 mittels eines Filmscharniers 23 mit dem Aufnahmeteil 21 verbunden und weist eine Rastnase 28a auf, welche zum Verschließen in eine entsprechende Ausnehmung 28b im Gehäuseteil einrasten kann. Eine Öse 24 kann z.B. zur Befestigung an einem Schlüsselbund dienen.

Der Aufnahmeteil 21 weist zwei längliche Aufnahmen 25, 26 auf, welche an die beiden länglichen Speicherabschnitte 2, 3 das aufzunehmenden Inhalationsgeräts 1 angepasst sind. Insbesondere kommt die jeweilige Randwulst 14, 15 mit einer umfangsseitigen Innenwand in einem Endbereich 25a, 26a der Aufnahmen 25, 26 in Kontakt, sodass die Hohlräume 9, 10 (für alle oben beschriebenen Ausführungsbeispiele des Inhalationsgeräts) gegen die Umgebung abgedichtet werden und die Wirkstoffe im Vergleich zu einer offenen Aufbewahrung länger erhalten bleiben. Die Randwulste 14, 15 können zudem zur weiteren Abdichtung bis an das Ende der Aufnahmen 25, 26 gedrückt werden, wenn das Behältnis 20 geschlossen wird. Dazu können in dem Deckel 22 entsprechende Strukturen 27 vorgesehen sein, welche im geschlossenen Zustand gegen den Bügelabschnitt 4 des Inhalationsgeräts 1 drücken. Die Aufnahmen 25, 26 weisen zudem entsprechende Aufnahmebereiche 25b, 26b für die Klemmvorsprünge 12, 13 auf.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Miniaturisiertes Inhalationsgerät (1) zum Inhalieren mindestens eines Wirkstoffs durch die Nase, mit einer im Wesentlichen U-förmigen Bauteilform und
- einem ersten länglichen Speicherabschnitt (2), welcher einen ersten Hohlraum (9) zum Aufnehmen einer ersten Menge eines Wirkstoffs aufweist und eingerichtet ist, in ein erstes Nasenloch eingeführt zu werden, wobei der erste Hohlraum (9) eine erste deckseitige Öffnung (2c) aufweist, über die der Wirkstoff aus dem ersten Hohlraum (9) austreten kann;
- einem zweiten länglichen Speicherabschnitt (3), welcher einen zweiten, von dem ersten Hohlraum (9) getrennten Hohlraum (10) zum Aufnehmen einer zweiten Menge des Wirkstoffs oder eines anderen Wirkstoffs aufweist und eingerichtet ist, in ein zweites Nasenloch eingeführt zu werden, wobei der zweite Hohlraum (10) eine zweite deckseitige Öffnung (3c) aufweist, über die der Wirkstoff und / oder der andere Wirkstoff aus dem zweiten Hohlraum (10) austreten kann;
- einem gekrümmten Bügelabschnitt (4), welcher den ersten und den zweiten länglichen Speicherabschnitt (2, 3) einstückig verbindet und sich zumindest in einem Fußbereich der U-förmigen Bauteilform erstreckt; und
- einem Selbsthaltemechanismus zum Halten an der Nase, bei dem der erste und der zweite längliche Speicherabschnitt (2, 3) mittels des gekrümmten Bügelabschnitts (4) gegen ein den Abstand zwischen dem ersten und dem zweiten länglichen Speicherabschnitt (2, 3) vergrößerndes Aufbiegen elastisch vorgespannt sind und im Bereich des ersten und des zweiten länglichen Speicherabschnitts (2, 3) jeweils innenseitig ein einstückig angeformter Klemmvorsprung (12, 13) angeordnet ist, welche zum Halten auf gegenüberliegenden Seiten der Nasenscheidewand zur Anlage kommen.

2. Miniaturisiertes Inhalationsgerät (1) nach Anspruch 1, **dadurch gekennzeichnet, dass** der erste und der zweite längliche Speicherabschnitt (2, 3) sowie der gekrümmte Bügelabschnitt (4) einen einstückigen Bauteilkörper mit der u-förmigen Bauteilform bilden, welcher aus einem Kunststoffmaterial besteht.

3. Miniaturisiertes Inhalationsgerät (1) nach Anspruch 1 oder 2, **dadurch gekennzeichnet, dass** der gekrümmte Bügelabschnitt (4) im Querschnitt flach ausgebildet ist.

4. Miniaturisiertes Inhalationsgerät (1) nach mindestens einem der vorangehenden Ansprüche, **dadurch gekennzeichnet, dass** der gekrümmte Bügelabschnitt (4) an den ersten und den zweiten längliche Speicherabschnitt (2, 3) jeweils innenseitig einstückig angeformt ist.

5. Miniaturisiertes Inhalationsgerät (1) nach mindestens einem der vorangehenden Ansprüche, **dadurch gekennzeichnet, dass** der erste und der zweite längliche Speicherabschnitt (2, 3) jeweils eine Zylinderform aufweisen.

6. Miniaturisiertes Inhalationsgerät (1) nach mindestens einem der vorangehenden Ansprüche, **dadurch gekennzeichnet, dass** der erste und der zweite Hohlraum (9, 10) jeweils eine Zylinderform aufweisen.

7. Miniaturisiertes Inhalationsgerät (1) nach mindestens einem der vorangehenden Ansprüche, **dadurch gekennzeichnet, dass** bei dem jeweiligen Klemmvorsprung (12, 13) eine Vorsprungspitze (12a, 13a) abgerundet ist.

8. Miniaturisiertes Inhalationsgerät (1) nach mindestens einem der vorangehenden Ansprüche, **dadurch gekennzeichnet, dass** der jeweilige Klemmvorsprung (12, 13) mit Blick von vorn auf die u-förmige Bauteilform eine Dreieckform aufweist.

9. Miniaturisiertes Inhalationsgerät (1) nach mindestens einem der vorangehenden Ansprüche, **dadurch gekennzeichnet, dass** der jeweilige Klemmvorsprung (12, 13) in Längsrichtung des ersten / zweiten länglichen Speicherabschnitts (2, 3) mit diesem vollständig überlappend gebildet ist.

10. Miniaturisiertes Inhalationsgerät (1) nach mindestens einem der vorangehenden Ansprüche, **dadurch gekennzeichnet, dass** der jeweilige Klemmvorsprung (12, 13) mit Blick von oben auf die u-förmige Bauteilform eine geringere Bauteildicke aufweist als der erste / zweite längliche Speicherabschnitt (2, 3).

11. Miniaturisiertes Inhalationsgerät (1) nach mindestens einem der vorangehenden Ansprüche, **dadurch gekennzeichnet, dass** der jeweilige Klemmvorsprung (12, 13) unterhalb und beabstandet von einem umlaufenden Rand der ersten / zweiten deckseitigen Öffnung (2c, 3c) angeordnet ist.

12. Miniaturisiertes Inhalationsgerät (1) nach mindestens einem der vorangehenden Ansprüche, **dadurch gekennzeichnet, dass**
- der erste Hohlraum (9) mit einem ersten Verschluss (7) aus für den Wirkstoff durchlässigem Material versehen ist und
- der zweite Hohlraum (10) mit einem zweiten Verschluss (8) aus für den Wirkstoff und / oder den anderen Wirkstoff durchlässigem Material versehen ist;
und / oder
in dem ersten und / oder dem zweiten Hohlraum (9, 10) ein Stopfen abgeordnet ist, in welchem der Wirkstoff und / oder der andere Wirkstoff abgebbar aufgenommen sind.

13. Miniaturisiertes Inhalationsgerät (1) nach mindestens einem der vorangehenden Ansprüche, **dadurch gekennzeichnet, dass** der Selbsthaltemechanismus entlang des umlaufenden Randes der ersten und der zweiten deckseitigen Öffnung jeweils eine außenseitige Randwulst (14, 15) aufweist.

14. Miniaturisiertes Inhalationsgerät (1) nach mindestens einem der vorangehenden Ansprüche, **dadurch gekennzeichnet, dass** die erste und die zweite deckseitige Öffnung (2c, 3c) einen Öffnungsquerschnitt aufweisen, welcher im Wesentlichen gleich einem Hohlraumquerschnitt im Bereich des ersten und des zweiten Hohlraums (9, 10) ist.

15. Set, umfassend mindestens ein miniaturisiertes Inhalationsgerät (1) nach mindestens einem der vorangehenden Ansprüche sowie ein Aufbewahrungsbehältnis (20) für das zumindest eine Inhalationsgerät (1), **dadurch gekennzeichnet, dass** das Aufbewahrungsbehältnis (20) eingerichtet ist, das Inhalationsgerät (1) aufzunehmen, wobei das Aufbewahrungsbehältnis (20) eine erste Aufnahme (25) und eine zweite Aufnahme (26) aufweist, welche bemessen und geformt sind, den ersten länglichen Speicherabschnitt (2) bzw. den zweiten länglichen Speicherabschnitt (3) jeweils zumindest teilweise darin aufzunehmen, sodass der erste und zweite Hohlraum (9, 10) gegenüber der Umgebung abgedichtet sind.
